# EUROPEAN PATENT SPECIFICATION

(11) **EP 4 335 915 B1**
(45) Date of publication and mention of the grant of the patent: **19.08.2026**
(21) Application number: 22841809.1
(22) Date of filing: 25.05.2022
(51) Int. Cl.: C12M 1/12, A61L 2/10, C12M 1/38

(54) **CULTURE APPARATUS**
KULTURVORRICHTUNG
APPAREIL DE CULTURE

(30) Priority: 15.07.2021 JP 2021117005
(43) Date of publication of application: 13.03.2024
(73) Proprietor: PHC Holdings Corporation, Tokyo 100-8403 (JP)
(72) Inventor: HORIUCHI, Kenichi, Toon-shi, Ehime 791-0395 (JP); HONDA, Kousuke, Toon-shi, Ehime 791-0395 (JP); HORIMOTO, Nobuo, Toon-shi, Ehime 791-0395 (JP); HIRAI, Hiroki, Toon-shi, Ehime 791-0395 (JP); OSHIMOTO, Taiki, Toon-shi, Ehime 791-0395 (JP); SAKAI, Yuta, Toon-shi, Ehime 791-0395 (JP); YOSHIDA, Kaori, Toon-shi, Ehime 791-0395 (JP)
(74) Representative: Epping - Hermann - Fischer
(86) International application number: PCT/JP2022/021426
(87) International publication number: WO 2023/286462

(56) References cited:
- EP-A1- 2 319 911
- WO-A1-2010/150782
- WO-A1-2011/031167
- WO-A1-2011/031167
- WO-A1-2018/061934
- CN-U- 212 116 492
- JP-A- 2015 026 901
- US-A1- 2020 093 945

## Description

### Technical Field

The present disclosure relates to culture systems.

### Background Art

In a culture apparatus (incubator) for incubating a culture such as a cell or a microorganism, a culture space is sterilized. An UV lamp is exemplified as a device for sterilization (see, for example, Patent Literature (hereinafter, referred to as "PTL") 1).
Document WO 2011/031167 A1 describes a module provided with a light emitting diode (LED) suitable to emit an ultraviolet ray and a control device including a memory that stores information related to the operation of the LED. The LED and the control device are connected to a power source.
Document EP 2 319 911 A1 describes a culture device comprising a closed enclosure provided with a light emitting diode (LED) suitable to emit an ultraviolet ray. The device further includes means for maintaining environmental conditions within the enclosure, and control means configured to control the operation of the enclosure, including the LED, using information stored in the controlling system.

### Citation List

### Patent Literature

PTL 1
Japanese Unexamined Patent Application Publication (Translation of PCT Application) No. 2018-512889

### Summary of Invention

### Technical Problem

Some UV lamps contain mercury. Recently, the use of mercury has been restricted. Under such circumstances, an object of the present disclosure is to provide a culture system which can sterilize a culture space of the culture apparatus by a novel device.

### Solution to Problem

An aspect of a culture system according to the present disclosure includes: a light emitting diode (LED) module including an LED that emits an ultraviolet ray, an information holding apparatus, and a module-side connector electrically connected to the LED and the information holding apparatus; and a culture apparatus including a box, an apparatus-side connector attached to the box and to which the module-side connector is connected, an atmosphere adjustment apparatus that is configured to adjust an atmosphere inside the box, and a control apparatus that is configured to control the atmosphere adjustment apparatus, in which the LED module can be detachably attached to the culture apparatus (1), and the control apparatus is configured to control the LED based on information acquired from the information holding apparatus via the apparatus-side connector and the module-side connector.

### Advantageous Effects of Invention

According to the present disclosure, it is possible to provide a culture system which can sterilize a culture space of the culture apparatus by a novel device.

### Brief Description of Drawings

FIG. 1 is a schematic diagram of a culture system according to an embodiment of the present disclosure;
FIG. 2 is a schematic longitudinal section of a culture apparatus according to an embodiment of the present disclosure viewed from the right side;
FIG. 3 is a perspective view of an LED module;
FIG. 4 is a perspective view of the LED module attached to a box;
FIG. 5 is a longitudinal sectional view of the LED module attached on the box and its surroundings;
FIG. 6 is a longitudinal sectional view of a dummy module attached to the box and its surroundings; and
FIG. 7 is a flowchart of an operation example of the culture apparatus;

### Description of Embodiments

Hereinafter, a culture apparatus according to an embodiment of the present disclosure will be described with reference to the drawings.

In the present specification, the front, rear, left, and right sides of the culture apparatus are defined as follows. That is, the side of the culture apparatus which the user faces during usage of the culture apparatus (the side with below-described outer door 3a and inner door 3b) is referred to as "front" and the side opposite to the front is referred to as "rear." In addition, the left and right are defined with reference to the case of viewing from the front to the rear.

Note that, in all the figures for explaining the embodiments, the same elements are denoted by the same reference numerals in principle, and the description thereof may be omitted.

### [1. Overall System Configuration]

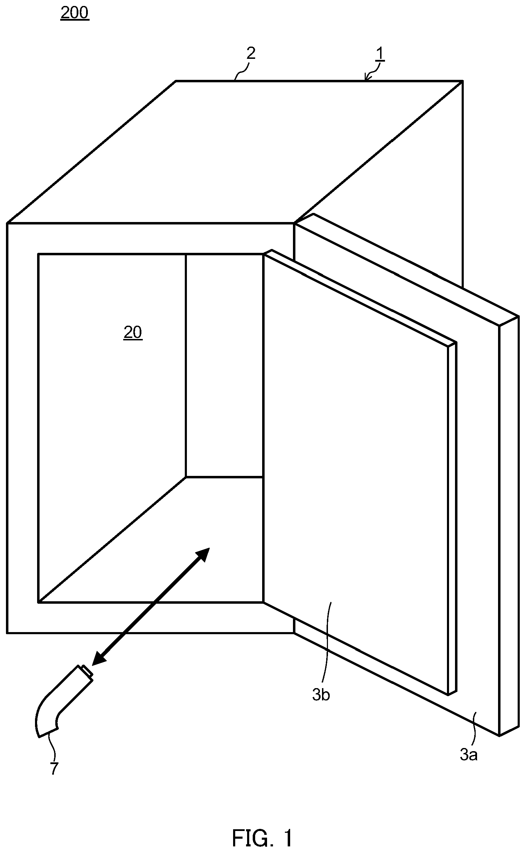

FIG. 1 is a schematic diagram of a culture system according to an embodiment of the present disclosure. Culture system 200 illustrated in FIG. 1 includes culture apparatus 1 and light emitting diode (LED) module 7. In FIG. 1, the internal details of culture space 20 of culture apparatus 1 are omitted. LED module 7 can be detachably attached to culture apparatus 1 from the inside of culture apparatus 1 (from the culture space 20 side described later). The arrows in FIG. 1 illustrate attachment of LED module 7 to culture apparatus 1 and removal of LED module 7 from culture apparatus 1. When the lifetime of LED module 7 is reached or LED module 7 is deteriorated, LED module 7 currently attached is removed from culture apparatus 1, and another LED module 7 is attached to culture apparatus 1. LED module 7 which has a desired performance may be selected from among a plurality of types of LED modules 7 as LED module 7 to be attached to culture apparatus 1. Culture apparatus 1 and LED module 7 will be described in detail later.

### [2. General Apparatus Configuration]

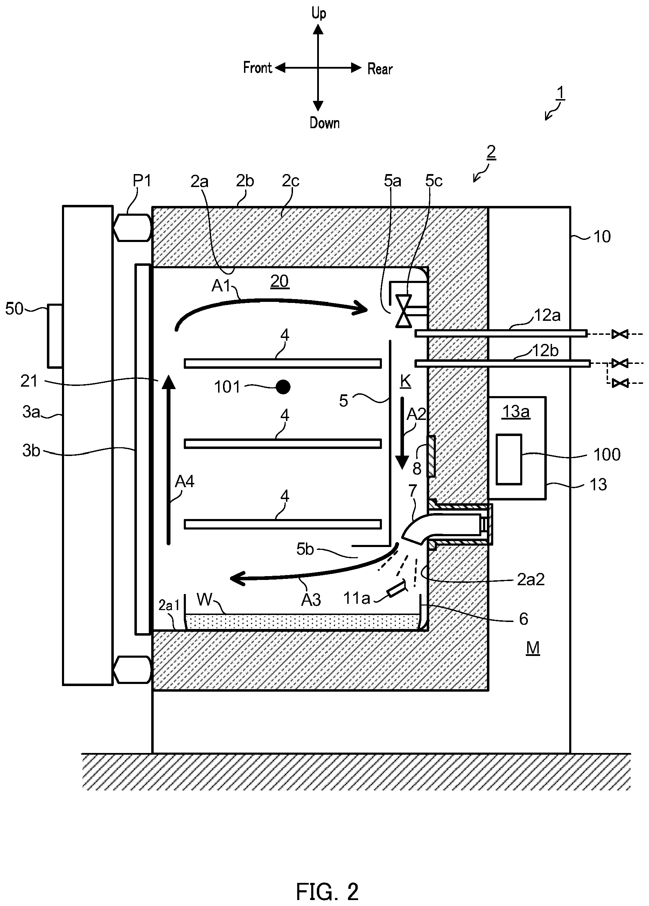

FIG. 2 is a schematic longitudinal section of culture apparatus 1 viewed from the right side.

Culture apparatus 1 illustrated in FIG. 2 is an apparatus for incubating a culture such as a cell or a microorganism. Culture apparatus 1 includes: substantially box-shaped box 2 having culture space 20 formed therein and opening 21 formed in a front surface thereof; and outer door 3a and inner door 3b for opening and closing opening 21. Culture space 20 is vertically compartmentalized by a plurality of shelves 4. Packing P1 is disposed on the outer edge of outer door 3a.

In culture space 20, the temperature, the humidity, O₂ (oxygen) concentration, and CO₂ (carbon dioxide) concentration are maintained in appropriate ranges such that an appropriate atmosphere for incubating the culture is achieved. Culture apparatus 1 includes temperature sensor 101 that detects the temperature in culture space 20.

Box 2 includes substantially box-shaped inner box 2a having culture space 20 formed therein, and substantially box-shaped outer box 2b covering the outside of inner box 2a.

Inner box 2a and outer box 2b are formed of a metallic plate. Heat insulation material 2c is disposed between inner box 2a and outer box 2b. Heat insulation material 2c is formed, for example, by combining plate-shaped heat insulation materials. A space (so-called air jacket) may be formed between inner box 2a and heat insulation material 2c.

In culture space 20, vertically extending duct 5 is disposed on the rear surface of inner box 2a. Gas passage K is formed inside duct 5. Circulation fan 5c is disposed in gas passage K. By operating circulation fan 5c, air in culture space 20 is sucked through suction port 5a formed in an upper portion of duct 5, and this air is blown out to culture space 20 through blow-out port 5b formed in a lower portion of duct 5. Thus, forced circulation of the air as indicated by arrows A1, A2, A3, and A4 takes place.

Humidification tray 6 for storing water W for humidification (hereinafter referred to as "humidification water W") is installed between the lower portion of duct 5 and bottom wall 2a1 of inner box 2a.

LED module 7 and gas supply apparatuses 12a and 12b for supplying culture space 20 with an adjustment gas for adjusting O₂ gas concentration and CO₂ gas concentration in culture space 20 (O₂ gas, N₂ gas, and CO₂ gas) are installed within duct 5. LED module 7 sterilizes water W in below-described humidification tray 6 and the air in culture space 20 by emitting ultraviolet rays. LED module 7 will be described in detail later.

In addition, heater 8 for temperature adjustment, that is, for controlling the temperature in culture space 20, is installed on each rear surface (surface on the side of outer box 2b) of the right side wall, the left side wall, rear wall 2a2, the top wall, and bottom wall 2a1 of inner box 2a. Note that heater 8 is being energized and generating heat, in principle, during the operation of culture apparatus 1.

The output (heating force) of heater 8 is controlled by control apparatus 100.

Circulation fan 5c, gas supply apparatuses 12a and 12b, and heater 8 constitute an atmosphere adjustment apparatus. The atmosphere adjustment apparatus is an apparatus that achieves an atmosphere suitable for incubating a culture inside box 2 (culture space 20). It is needless to say that the atmosphere adjustment apparatus may be constituted by other elements in addition to circulation fan 5c, gas supply apparatus 12a and 12b, and heater 8.

In addition, culture apparatus 1 receives instructions to start and stop culture apparatus 1, operation mode settings, and inputs of various setting values for culture space 20 from manipulation apparatus 50 disposed on outer door 3a. The various setting values for culture space 20 include a set temperature, set humidity, set concentration of O₂ gas, set concentration of CO₂ gas, and/or the like. Control apparatus 100 controls the components such as the atmosphere adjustment apparatus and LED module 7 based on the input from manipulation apparatus 50. Manipulation apparatus 50 includes a display section that displays the state of culture apparatus 1.

The operation modes of culture apparatus 1 includes at least a normal operation mode and a dry heat sterilization mode. The normal operation mode is a mode in which the atmosphere adjustment apparatus is operated so that an atmosphere suitable for incubating the culture (for example, 37°C) is achieved in the inside of box 2 (culture space 20). Further, the dry heat sterilization mode is a mode in which the atmosphere adjustment apparatus is operated so as to dry heat sterilize the inside of box 2 (culture space 20). When dry heat sterilization is performed, humidification tray 6 is emptied, and the inside of box 2 (culture space 20) is maintained at, for example, 180°C.

The back surface and the bottom surface of outer box 2b of box 2 are covered with cover 10. The space between the back surface of outer box 2b and cover 10 forms mechanical room M for disposing various equipment therein. Electrical box 13 is disposed in mechanical room M. Control apparatus 100 and other electrical components (not illustrated) are housed in inside 13a of electrical box 13.

Further, the tip of dew condensation member 11a is inserted into culture space 20. Dew condensation member 11a is cooled by a Peltier element (not illustrated). Accordingly, condensation water is generated on the surface of dew condensation member 11a in culture space 20. Generation of the condensation water makes it possible to reduce the humidity in culture space 20 to control the humidity within an appropriate range. Note that, the condensation water generated on the surface of dew condensation member 11a drips from the tip of dew condensation member 11a into humidification tray 6.

### [3. LED Module]

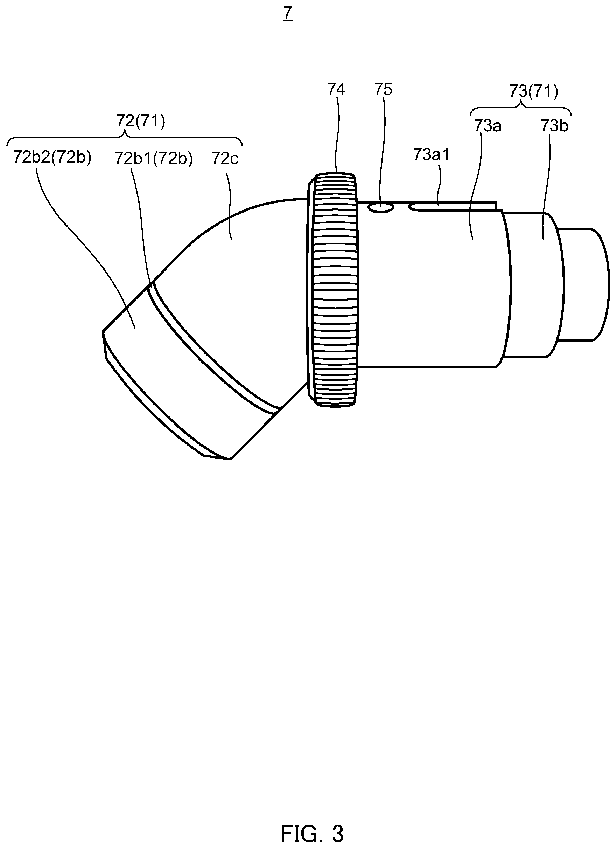

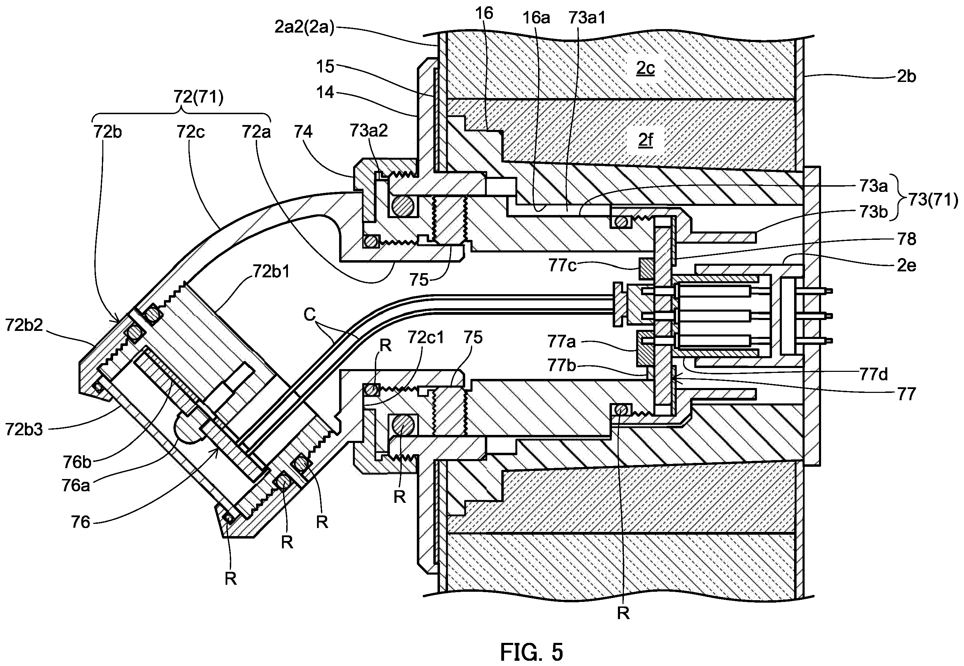

FIG. 3 is a perspective view of LED module 7. LED module 7 includes LED 76a (see FIG. 5) to be described later, metallic cylindrical body 71 in which LED 76a is accommodated, and metallic coupling 74.

Cylindrical body 71 includes distal-end-side cylindrical body 72 and proximal-end-side cylindrical body 73. Groove 73a1 extending parallel to the central axis of the proximal-end-side cylindrical body is formed in the outer peripheral surface of proximal-end-side cylindrical body 73. Further, a through-hole which is a female screw is formed in the outer peripheral surface of proximal-end-side cylindrical body 73, and rotation restriction member 75 having a male screw shape is inserted into the through-hole. Groove 73a1 and rotation restriction member 75 will be described in detail later.

Coupling 74 is a ring-shaped member surrounding cylindrical body 71 and is relatively rotatable with respect to cylindrical body 71.

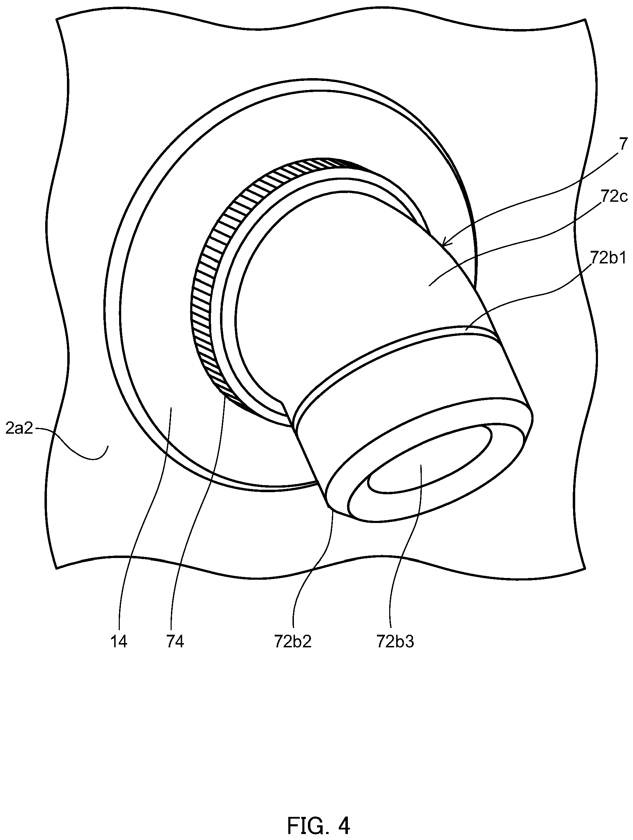

FIG. 4 is a perspective view of LED module 7 attached to box 2 as viewed from culture space 20. An insertion opening which opens at least toward the culture space 20 side is formed in box 2, and LED module 7 is inserted into and fixed to the insertion opening. Flange-shaped engaged portion 14 that rims the insertion opening is fixed by, for example, a plurality of bolts on rear wall 2a2 of inner box 2a facing culture space 20. Seal 15 is sandwiched between engaged portion 14 and rear wall 2a2. Engaged portion 14 has a male screw portion, and coupling 74 has a female screw portion. By connecting the male screw portion and the female screw portion to each other, LED module 7 is fixed to box 2.

The connection between coupling 74 and engaged portion 14 can be realized by various known connection means, such as a bayonet or a quick joint, instead of a screw. The bayonet is, for example, a connection means used in an interchangeable lens attachment structure of a single-lens reflex camera. That is, when the bayonet is applied to the present embodiment, engaged portion 14 includes a plate-like portion in which a plurality of holes or grooves are formed, a plurality of engaging pieces are formed on coupling 74, and coupling 74 may be connected to engaged portion 14 after the engaging pieces are inserted into the holes or grooves and coupling 74 rotates about the central axis of coupling 74 to move to the back side of the plate-like portion.

FIG. 5 is a longitudinal sectional view of LED module 7 attached to box 2 and its surroundings.

The insertion opening in box 2 into which LED module 7 is inserted is formed by resin-made sleeve 16. Sleeve 16 is disposed between inner box 2a and outer box 2b. Sleeve 16 is disposed between inner box 2a and outer box 2b, and is disposed in cylindrical heat insulation material 2f defining the opening in box 2. Guide rail 16a protruding toward the inner surface side is formed on the inner surface of sleeve 16.

The insertion opening in box 2 into which LED module 7 is inserted is naturally a space free of heat insulation material 2c. Therefore, this portion has a lower heat insulation property, and is likely to serve as a heat passage between the outside of box 2 and the inside (culture space 20) of box 2. However, by making sleeve 16 from resin, it is possible to reduce the ease of heat transfer between the inside and the outside of box 2 (specifically, between inner box 2a and outer box 2b and between the inside and the outside of outer box 2b) in the vicinity of the insertion opening into which LED module 7 is inserted.

Apparatus-side connector 2e is attached to outer box 2b. Apparatus-side connector 2e is disposed in the insertion opening into which LED module 7 is inserted, in particular in sleeve 16. Apparatus-side connector 2e is disposed at a position closer to outer box 2b than inner box 2a, specifically, at a position between the inner end face and the outer end face of outer box 2b and closer to the outer end face. In other words, apparatus-side connector 2e is disposed at a position that is not easily affected by the temperature in culture space 20.

Cylindrical body 71 constituting LED module 7 includes metallic distal-end-side cylindrical body 72, and metallic proximal-end-side cylindrical body 73 disposed on the proximal end side of distal-end-side cylindrical body 72. Proximal-end-side cylindrical body 73 includes socket joint 73a and end cap 73b, which will be described in detail below. Specifically, the through-hole into which above-described rotation restriction member 75 (see FIG. 2) is inserted is formed in socket joint 73a.

Distal-end-side cylindrical body 72 is coaxial with socket joint 73a and includes connection portion 72a connected to socket joint 73a, LED accommodation portion 72b inclined with respect to connection portion 72a and accommodating LED 76a, and elbow portion 72c connecting between connection portion 72a and LED accommodation portion 72b. In the present embodiment, connection portion 72a and elbow portion 72c are formed of one piece, but connection portion 72a and elbow portion 72c may be formed of respective separate components.

LED accommodation portion 72b includes LED mounting body 72b1 and front cap 72b2. LED mounting body 72b1 includes a relatively thick solid portion, and distal-end-side board 76 to which LED 76a is attached is disposed on a distal end side of the solid portion. Thermally conductive sheet 76b is disposed between LED mounting body 72b1 and distal-end-side board 76. Therefore, the heat generated by LED 76a is efficiently transmitted to the relatively thick solid portion via thermally conductive sheet 76b. The relatively thick solid portion serves as a kind of heat sink.

A male screw portion is formed on a distal end side of LED mounting body 72b1, and a female screw portion is formed on an inner surface side of front cap 72b2. By connecting these screw portions to each other, LED mounting body 72b1 and front cap 72b2 are connected to each other. At this time, window 72b3 and O-ring R is sandwiched between the distal end of LED mounting body 72b1 and the distal-end-side inner surface of front cap 72b2, and O-ring R is sandwiched between the proximal-end-side inner surface of front cap 72b2 and LED mounting body 72b1.

A male screw portion is formed on the proximal-end-side outer peripheral surface of LED mounting body 72b1, and a female screw portion is formed on the inner peripheral surface of elbow portion 72c. By connecting these screw portions to each other, elbow portion 72c is connected to LED mounting body 72b1 and thus to LED accommodation portion 72b.

A male screw portion is formed on an outer peripheral surface of connection portion 72a integrally formed with elbow portion 72c, and a female screw portion is formed on a distal-end-side inner peripheral surface of socket joint 73a. By connecting the male screw portion and the female screw portion to each other, distal-end-side cylindrical body 72 and socket joint 73a are connected to each other. At this time, O-ring R is sandwiched between the distal-end-side inner peripheral surface of socket joint 73a and the outer peripheral surface of connection portion 72a.

Rotation restriction member 75 is inserted into socket joint 73a. In addition, a flat surface portion is formed on a part of the outer peripheral surface of connection portion 72a. When the inner side of a tip end of rotation restriction member 75 makes contact with the flat surface portion, distal-end-side cylindrical body 72 and socket joint 73a are prevented from relatively rotating with respect to each other. That is, the attitude of distal-end-side cylindrical body 72 with respect to proximal-end-side cylindrical body 73 (circumferential angle, and thus, the direction in which LED accommodation portion 72b faces) can be set to a particular attitude (the direction in which LED accommodation portion 72b faces humidification tray 6). When the frictional force acting between rotation restriction member 75 and connection portion 72a can be sufficiently increased, the flat surface portion is not formed on the outer peripheral surface of connection portion 72a, and the entire outer peripheral surface of connection portion 72a may be a cylindrical surface.

Further, rotation restriction member 75 is in close contact with socket joint 73a and connection portion 72a. Therefore, although LED 76a generates heat as described later, rotation restriction member 75 can transmit the heat transmitted from LED 76a to distal-end-side cylindrical body 72 to proximal-end-side cylindrical body 73. That is, by attaching rotation restriction member 75, it is possible to increase a path through which the heat generated by LED 76a is released, and to dissipate the heat more efficiently.

A male screw portion is formed on a proximal end side of socket joint 73a, and a female screw portion is formed on an inner surface side of end cap 73b. By connecting these screw portions to each other, socket joint 73a and end cap 73b are connected to each other. At this time, proximal-end-side board 77 and seal 78 are sandwiched between a proximal-end-side step portion of socket joint 73a and a step portion of end cap 73b, and O-ring R is sandwiched between the distal-end-side inner surface of end cap 73b and socket joint 73a.

Module-side connector 77d is attached to the proximal-end-side surface of proximal-end-side board 77, that is, a surface facing the outer side of cylindrical body 71. When LED module 7 is inserted into sleeve 16 in a state where guide rail 16a formed on the inner surface of sleeve 16 is positioned in groove 73a1 formed in the outer surface of socket joint 73a, the position and orientation of module-side connector 77d are set so that module-side connector 77d is fitted to apparatus-side connector 2e.

Various components electrically connected to module-side connector 77d are attached to the distal-end-side surface of proximal-end-side board 77, that is, to the surface facing the inside of cylindrical body 71. One of the components is cable C that connects proximal-end-side board 77 to distal-end-side board 76 and supplies power to LED 76a. Heater 77a may be attached to proximal-end-side board 77. In addition, metallic foil pattern 77b may be formed on the surface of proximal-end-side board 77. Further, information holding apparatus 77c is attached to proximal-end-side board 77. Information holding apparatus 77c may be any apparatus capable of holding and outputting predetermined information, and is, for example, a semiconductor memory or a DIP switch. As will be described later, when control apparatus 100 acquires the cumulative lighting time from information holding apparatus 77c, it is preferable that information holding apparatus 77c include a semiconductor memory to or from which information can be written or read. In addition, control apparatus 100 is configured to be able to store, in information holding apparatus 77c, the duration over which LED 76a is on.

Coupling 74 includes a flange-shaped portion and a cylindrical portion connected to an outer peripheral end portion of the flange-shaped portion. The flange-shaped portion is located between proximal-end-side end face 72c1 of elbow portion 72c and flange portion 73a2 formed on the distal end side of socket joint 73a. Further, a female screw portion is formed on an inner surface of the cylindrical portion. The female screw portion is connected to a male screw portion formed so as to protrude toward the distal end side of engaged portion 14.

When the female screw portion of coupling 74 is not connected to the male screw portion of engaged portion 14, there is a gap between, on one hand, the flange-shaped portion of coupling 74 and, on the other hand, elbow portion 72c and flange portion 73a2. Thus, coupling 74 can freely rotate with respect to cylindrical body 71.

When coupling 74 is rotated after cylindrical body 71 is inserted into sleeve 16, the female screw portion of coupling 74 and the male screw portion of engaged portion 14 can be connected to each other. At this time, O-ring R is sandwiched between the outer peripheral surface of socket joint 73a and the inner peripheral surface of engaged portion 14. At this time, flange portion 73a2 makes contact with coupling 74 and engaged portion 14, and is relatively strongly sandwiched between coupling 74 and engaged portion 14. That is, cylindrical body 71 is in close contact with coupling 74 and engaged portion 14. Therefore, as will be described later, the heat transmitted to cylindrical body 71 is rapidly transmitted to inner box 2a via engaged portion 14.

When the female screw portion of coupling 74 and the male screw portion of engaged portion 14 are connected to each other, the inner peripheral surface of the cylindrical portion of engaged portion 14 and the outer peripheral surface of socket joint 73a are close to each other. Further, the distal end surface of socket joint 73a and the proximal-end-side end face 72c1 of elbow portion 72c are close to each other. Therefore, as will be described later, the heat generated by LED 76a is rapidly transmitted to inner box 2a via cylindrical body 71 and engaged portion 14.

When such an LED module 7 is attached to box 2, control apparatus 100 acquires predetermined information from information holding apparatus 77c via apparatus-side connector 2e and module-side connector 77d. Examples of the predetermined information include information indicating that an apparatus attached to box 2 is LED module 7, or identification information allowing identification of at least one of LED 76a and LED module 7. Specific examples of the identification information include a model identification number, an individual identification number, and the like of LED 76a or LED module 7. By acquiring the predetermined information, control apparatus 100 can recognize that LED module 7 is attached. Thus, control apparatus 100 can cause the culture apparatus to incubate the culture while sterilizing the culture by emitting ultraviolet rays from LED 76a into culture space 20, that is, to operate in the normal operation mode.

In addition, when the predetermined information is identification information, control apparatus 100 can control LED 76a under appropriate conditions according to the type of LED 76a included in LED module 7, as a result of acquiring the identification information. For example, an appropriate supply current value and a length of time for supplying the current may be determined based on the type of LED 76a, and LED 76a may be on at the determined supply current value and for the determined supply time. For example, in LED module 7 including LED 76a for which the rated current is large and the intensity of ultraviolet rays emitted is high, the sterilization of culture space 20 can be completed in a relatively short time. Therefore, the supply current value may be increased and the time for supplying the current may be shortened. On the other hand, in LED module 7 including LED 76a for which the rated current is small and the intensity of ultraviolet rays is low, it takes a relatively long time to complete the sterilization of culture space 20. Therefore, the supply current value may be reduced and the time for supplying the current may be increased. It is needless to say that control apparatus 100 may control not only both the length of time for lighting of LED 76a and the magnitude of the current supplied to LED 76a, but also one of them, based on the identification information.

The identification information may be acquired based on a combination of voltage values generated at predetermined two pins among a plurality of pins included in module-side connector 77d. The voltage value generated at each pin can be changed by adjusting the resistance value of each pin. That is, instead of information holding apparatus 77c which is, for example, a semiconductor memory or a DIP switch, two resistors respectively attached to predetermined two pins from among the plurality of pins included in module-side connector 77d may function as the information holding apparatus.

Control apparatus 100 may store combinations of two voltage values and identification information in association with each other in advance. Control apparatus 100 can acquire the identification information on LED module 7 based on the voltage values acquired from the predetermined two pins and the information stored in advance. Control apparatus 100 may acquire the voltage values of the two predetermined pins as analog values, and perform A/D conversion on the acquired analog values, to acquire the identification information based on the two converted values. By using the analog values, a large number of patterns of a combination of two voltage values can be created. In other words, only by changing the resistance values of the two pins, it is possible to generate various types of identification information without increasing the number of pins of module-side connector 77d. In addition, LED module 7 can be manufactured at a lower cost than when the semiconductor memory is used.

In addition, the predetermined information may be the cumulative lighting time of LED 76a. When acquiring the cumulative lighting time, control apparatus 100 can control LED 76a according to the state (for example, the degree of deterioration) of LED 76a included in LED module 7. For example, at least one of the appropriate supply current value and the length of time for supplying the current may be determined based on the state of LED 76a, and LED 76a may be turned on at the determined supply current value and for the supply time.

The deterioration of LED 76a progresses as the cumulative lighting time increases. When the deterioration progresses, less intense ultraviolet rays than those before the deterioration are emitted even when a current as large as that before the deterioration is supplied. In such a state, the sterilization effect is reduced. Therefore, control apparatus 100 increases the current to be supplied to LED 76a as the acquired cumulative lighting time increases. Thus, a decrease in the sterilization effect can be suppressed.

In addition, control apparatus 100 may store a first factor in advance in association with the cumulative lighting time of LED 76a, which first factor increases monotonically or in a stepwise manner with increasing cumulative lighting time of LED 76a. In this case, control apparatus 100 may determine the first factor based on the acquired cumulative lighting time, multiply a reference current value by the determined first factor, and determine a resultant value as the magnitude of the current to be supplied to LED 76a. Accordingly, LED 76a can be on at an appropriate current corresponding to the cumulative lighting time.

Further, as described above, instead that the current supplied to LED 76a is made larger, the duration over which LED 76a is on may be made longer as the acquired cumulative lighting time increases. Thus, a decrease in the sterilization effect can be suppressed.

In addition, control apparatus 100 may store a second factor in advance in association with the cumulative lighting time of LED 76a, which second factor increases monotonically or in a stepwise manner with increasing cumulative lighting time of LED 76a. In this case, control apparatus 100 may determine the second factor based on the acquired cumulative lighting time, multiply the reference time length by the determined second factor, and determine a resultant value as the length of time for lighting of LED 76a. Accordingly, LED 76a can be on for an appropriate lighting time corresponding to the cumulative lighting time.

In addition, LED 76a generates heat when ultraviolet rays are emitted, and is relatively vulnerable to heat. Then, the higher the temperature inside box 2 (that is, the temperature in culture space 20), the higher the temperature of LED 76a disposed in culture space 20. Therefore, as the temperature inside box 2 becomes higher, the heat generated by LED 76a is reduced. That is, by reducing the supply current value, an increase in the temperature of LED 76a can be suppressed, and thus the progress of the deterioration of LED 76a can be delayed. However, when the supply current value is reduced, the intensity of ultraviolet rays decreases and the sterilization effect decreases. Therefore, control apparatus 100 may reduce the current supplied to LED 76a and increase the duration for lighting of LED 76a as the temperature inside box 2 increases. Accordingly, the sterilization effect can be maintained, and culture space 20 can be reliably sterilized while the progress of the deterioration of LED 76a is made gentle. Control apparatus 100 acquires the temperature inside box 2 from temperature sensor 101. Note that control apparatus 100 may acquire, as the temperature inside box 2, the set temperature in culture space 20 input to manipulation apparatus 50.

In addition, control apparatus 100 may store a third factor in advance in association with the temperature inside box 2, which third factor decreases monotonically or in a stepwise manner with an increase in the temperature inside box 2. In this case, control apparatus 100 may determine the third factor based on the acquired temperature inside box 2, multiply a reference current value by the determined third factor, and determine a resultant value as the magnitude of the current to be supplied to LED 76a. Therefore, LED 76a can be on at an appropriate current corresponding to the temperature inside box 2.

In this case, control apparatus 100 may store a plurality of sets of the third factor and the temperature inside box 2 in advance, and may select the set to be used for controlling LED 76a based on the acquired identification information. By selecting an appropriate set based on the type of LED 76a, the progress of degradation of LED 76a can be reliably delayed by appropriately reducing the heat generated by LED 76a, i.e., by appropriately reducing the supply current value when the temperature inside box 2 is high.

In addition, control apparatus 100 may store a fourth factor in advance in association with the temperature inside box 2, which fourth factor increases monotonically or in a stepwise manner with increasing temperature inside box 2. In this case, control apparatus 100 may determine the fourth factor based on the acquired temperature inside box 2, multiply the reference time length by the determined fourth factor, and determine a resultant value as the length of time for lighting of LED 76a so as to maintain the sterilization effect correspondingly to the reduction in the supply current value. Accordingly, LED 76a can be on for an appropriate lighting time corresponding to the temperature inside box 2 such that the sterilization effect is maintained.

In addition, control apparatus 100 may store a plurality of sets of the fourth factor and the temperature inside box 2 in advance, and may select the set to be used for controlling LED 76a based on the acquired identification information. By selecting an appropriate set based on the type of LED 76a, it is possible to appropriately prevent a temperature rise in LED 76a and resulting accelerated deterioration, while maintaining the sterilization efficacy.

LED 76a generates heat simultaneously with emission of ultraviolet rays. Since LED 76a is relatively vulnerable to heat, the heat emitted from LED 76a must be rapidly dissipated to prevent LED 76a from becoming hot.

LED module 7 includes metallic cylindrical body 71 as described above. Therefore, the heat generated by LED 76a is easily transmitted to cylindrical body 71. Further, cylindrical body 71 is in contact with metallic engaged portion 14, and engaged portion 14 is in contact with metallic inner box 2a. Therefore, cylindrical body 71 and coupling 74 dissipate the heat generated by LED 76a to engaged portion 14. The heat transferred to engaged portion 14 is rapidly transferred to the metallic inner box. That is, the heat transferred from LED 76a to cylindrical body 71 is transferred to inner box 2a via engaged portion 14. Therefore, the heat generated by LED 76a can be efficiently dissipated through a heat transfer route formed by the metallic members, and a temperature rise in LED 76a can thus be prevented. Further, since resin-made sleeve 16 that is unlikely to transmit heat is disposed between inner box 2a and outer box 2b, it is possible to prevent the heat generated by LED 76a from being transmitted to apparatus-side connector 2e.

Due to formation of the heat transfer route as described above, it is possible to prevent LED 76a from failing or deteriorating due to the heat generated by itself under the temperature environment (for example, 37°C) in culture space 20 during operation in the normal operation mode. However, exposure to the temperature (e.g., 180°C) in culture space 20 during when the dry heat sterilization mode is performed may cause failure or deterioration. Therefore, when culture apparatus 1 is operated in the dry heat sterilization mode, it is preferable that LED module 7 be removed from box 2.

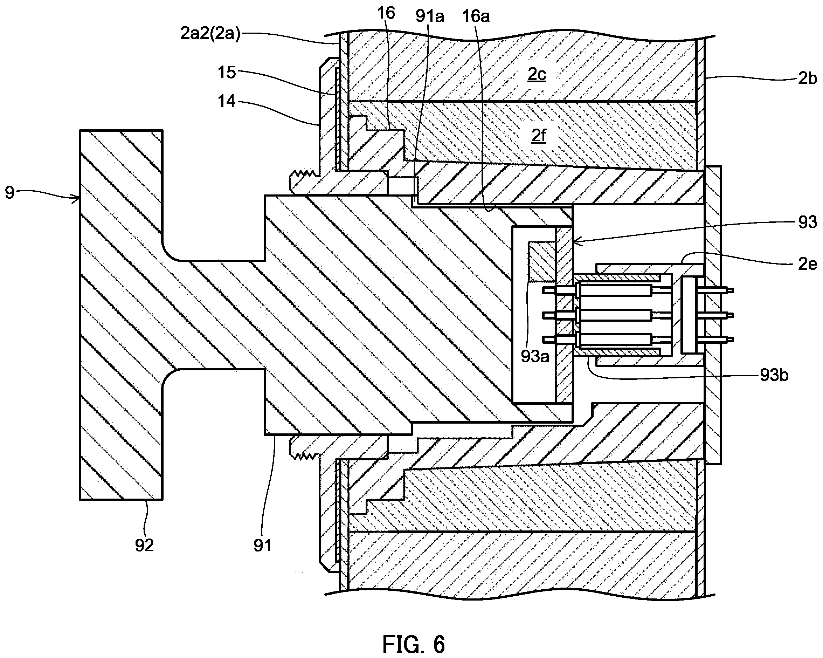

In this case, it is preferable that dummy module 9 illustrated in FIG. 6 instead of LED module 7 be attached to box 2. FIG. 6 is a longitudinal sectional view of dummy module 9 attached to box 2 and its surroundings. Dummy module 9 includes dummy main body 91 and handle 92 formed on a distal end side of dummy main body 91. Dummy module 9 can be attached to box 2 by inserting the dummy module into the insertion opening while holding handle 92. Groove 91a into which guide rail 16a can be inserted is formed in a side surface of dummy main body 91.

Dummy board 93 may be fixed to the proximal end side of dummy module 9. Dummy connector 93b may be attached to the proximal end side surface of dummy board 93, and information holding apparatus 93a electrically connected to dummy connector 93b may be attached to the front end side surface of dummy board 93. When dummy module 9 includes dummy board 93, dummy connector 93b, and information holding apparatus 93a, dummy connector 93b is connected to apparatus-side connector 2e and, accordingly, control apparatus 100 acquires predetermined information from information holding apparatus 93a via apparatus-side connector 2e and dummy connector 93b. The predetermined information is, for example, information indicating that the apparatus attached to box 2 is dummy module 9. By acquiring the predetermined information, control apparatus 100 can recognize that dummy module 9 is attached. When attachment of dummy module 9 is used as the condition for the operation in the dry heat sterilization mode, control apparatus 100 can cause the culture apparatus to operate in the dry heat sterilization mode while dummy module 9 is protecting apparatus-side connector 2e from heat.

That is, by attaching dummy module 9 to box 2, it is possible to prevent apparatus-side connector 2e from being directly exposed to high-temperature culture space 20 in the dry heat sterilization mode, and it is possible to prevent failure of apparatus-side connector 2e.

Further, apparatus-side connector 2e is disposed at a position closer to outer box 2b than to inner box 2a. Therefore, apparatus-side connector 2e is less likely to be affected by the temperature in culture space 20, in particular, the high temperature during when dry heat sterilization is performed. Moreover, the space in which dummy main body 91 is inserted can be enlarged, in other words, the volume of dummy main body 91 inserted into the insertion opening can be increased. Therefore, the heat insulation performance of dummy module 9, that is, the effect of protecting apparatus-side connector 2e from heat can be increased.

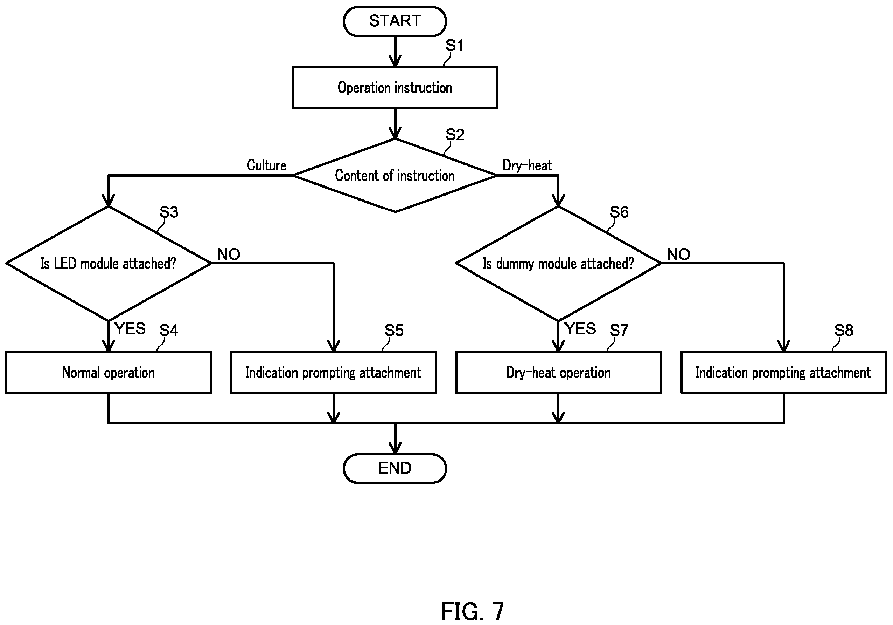

FIG. 7 is a flowchart illustrating an operation example of culture apparatus 1. Hereinafter, an operation example will be described with reference to FIG. 7.

When manipulation apparatus 50 is manipulated, control apparatus 100 receives an operation instruction (S1). Upon receiving the operation instruction, control apparatus 100 confirms the content of the instruction (S2). When the content of the instruction is an instruction for the culture operation ("Culture" in S2), control apparatus 100 checks whether or not LED module 7 is attached to box 2 (S3). When LED module 7 is attached (YES in S3), control apparatus 100 operates culture apparatus 1 in the normal operation mode by operating the atmosphere adjustment apparatus (circulation fan 5c, gas supply apparatuses 12a and 12b, heater 8, and the like) (S4).

At this time, control apparatus 100 acquires predetermined information from information holding apparatus 77c, and controls LED 76a based on the acquired predetermined information. Thus, the appropriate current and lighting time can be determined based on the type or status of LED module 7 or LED 76a. At this time, the temperature in culture space 20 may be detected by temperature sensor 101, and the current value to be supplied to LED 76a and the length of the time for supplying the current to LED 76a may be determined based on the detected temperature.

In addition, when the cumulative lighting time is acquired from information holding apparatus 77c, control apparatus 100 may calculate a new cumulative lighting time by adding the length of time for lighting of LED 76a, and store the calculated cumulative lighting time in information holding apparatus 77c. When the calculated cumulative lighting time exceeds a predetermined threshold, an indication prompting replacement of LED module 7 may be displayed on manipulation apparatus 50. The threshold is, for example, the length of the life over which LED 76a effectively functions, or the length resulting from subtraction of a predetermined period of time from the life. By performing such an indication, LED module 7 can be replaced at an appropriate timing, and culture space 20 can be sterilized appropriately at all times.

When LED module 7 is not attached (NO in S3), control apparatus 100 performs an indication prompting attachment of LED module 7 on the display section of manipulation apparatus 50 (S5). Therefore, it is possible to prevent an operation of incubating the culture from being performed in a state in which LED module 7 is not attached, that is, in a state in which sterilization by ultraviolet radiation cannot be performed.

When the received operation instruction is an instruction for the dry-heat operation ("Dry-heat" in S2), control apparatus 100 checks whether or not dummy module 9 is attached to box 2 (S6). When dummy module 9 is attached (YES in S6), control apparatus 100 causes culture apparatus 1 to operate in the dry-heat operation mode by operating the atmosphere adjustment apparatus (S7).

When dummy module 9 is not attached (NO in S6), control apparatus 100 performs an indication prompting attachment of dummy module 9 on the display section of manipulation apparatus 50 (S8). Therefore, it is possible to prevent the dry-heat operation from being performed in a state in which dummy module 9 is not attached, that is, in a state in which there is nothing to block between apparatus-side connector 2e and culture space 20.

LED module 7 includes metallic cylindrical body 71, and thus easily transmits heat. In addition, the insertion opening in box 2 into which LED module 7 is inserted is less thermally insulated. Therefore, when the outside air temperature is low, the temperature at the distal end side of LED module 7 may decrease, and thus the temperature in culture space 20 may be lowered. In order to prevent such a temperature drop, LED module 7 according to the present embodiment may include heater 77a on the surface of proximal-end-side board 77. When control apparatus 100 operates heater 77a, it is possible to prevent the temperature in culture space 20 from decreasing when the temperature around culture apparatus 1, that is, the outside air temperature, is low. In addition, LED module 7 according to the present embodiment may include metallic foil pattern 77b on the surface of proximal-end-side board 77. By providing metallic foil pattern 77b, heat generated by heater 77a can be efficiently transmitted to cylindrical body 71. Further, metallic foil pattern 77b may be in contact with cylindrical body 71, specifically, with proximal-end-side cylindrical body 73, more specifically, with the inner peripheral surface of socket joint 73a. Such contact allows heat to be transmitted more efficiently. Heater 77a may be attached to a location other than the surface of proximal-end-side board 77, for example, to the inner surface of socket joint 73a.

Further, culture apparatus 1 includes resin-made sleeve 16 disposed between inner box 2a and outer box 2b. Therefore, it is possible to suppress the heat transfer between the inside and outside the box, that is, to suppress the extent of decrease in the temperature in culture space 20 in the case where the outside air temperature is low.

As mentioned above, LED 76a generates heat. Therefore, when LED 76a is off, the temperature in culture space 20 decreases. Therefore, by operating heater 77a when LED 76a is off, it is possible to suppress a decrease in the temperature in culture space 20. Also, in this instance, the electric power supplied to heater 77a may be controlled such that the amount of heat given by LED 76a to inner box 2a during operation of LED 76a is substantially equal to the amount of heat given by heater 77a to inner box 2a during operation of heater 77a. By controlling the electric power in this way, it is possible to prevent a change in the temperature in culture space 20 due to the operation of LED 76a and heater 77a.

As described above, when the outside air temperature is low, the temperature at the distal end side of LED module 7 may decrease, and thus the temperature in culture space 20 may be lowered. By taking advantage of this property, when the outside air temperature is lower than the dew point of the inside of box 2, that is, culture space 20, dew condensation water may be generated on the distal-end-side surface of LED module 7. That is, LED module 7 may function as a member for adjusting the humidity in culture space 20 instead of dew condensation member 11a. In this case, the positions of LED module 7 and humidification tray 6 are set such that the dew condensation water generated on the distal-end-side surface of LED module 7 falls into humidification tray 6.

### Industrial Applicability

The present disclosure is suitably used as a culture system.

## Claims

1. A culture system (200), comprising:
a light emitting diode (LED) module (7) including:
an LED (76a) that is configured to emit an ultraviolet ray,
an information holding apparatus (77c), and
a module-side connector (77d) electrically connected to the LED (76a) and the information holding apparatus (77c); and
a culture apparatus (1) including:
a box (2),
an apparatus-side connector (2e) attached to the box (2) and to which the module-side connector (77d) is connected,
an atmosphere adjustment apparatus (5c, 8, 12a, 12b) that is configured to adjust an atmosphere inside the box (2), and
a control apparatus (100) that is configured to control the atmosphere adjustment apparatus (5c, 8, 12a, 12b),
**characterized in that**:
the LED module (7) can be detachably attached to the culture apparatus (1), and
the control apparatus (100) is configured to control the LED (76a) based on information acquired from the information holding apparatus (77c) via the apparatus-side connector (2e) and the module-side connector (77d).

2. The culture system (200) according to claim 1, wherein
the information includes identification information allowing identification of at least one of the LED (76a) or the LED module (77d).

3. The culture system (200) according to claim 2, wherein
based on the identification information acquired, the control apparatus (100) is configured to control at least one of a length of time for lighting of the LED (76a) or a magnitude of a current supplied to the LED (76a).

4. The culture system (200) according to any one of claims 1 to 3, wherein
the information includes a cumulative lighting time of the LED (76a).

5. The culture system (200) according to claim 4, wherein
based on the cumulative lighting time acquired, the control apparatus (100) is configured to control at least one of a length of time for lighting of the LED (76a) or a magnitude of a current supplied to the LED (76a).

6. The culture system (200) according to claim 5, wherein
the control apparatus (100) is configured to increase the current supplied to the LED (76a) or to increase the time for lighting of the LED (76a) as the cumulative lighting time acquired becomes longer.

7. The culture system (200) according to claim 6, wherein:
the control apparatus (100) is configured to store a first factor in association with the cumulative lighting time of the LED (76a), the first factor increasing monotonically or in a stepwise manner with an increase in the cumulative lighting time of the LED (76a), and
the control apparatus (100) is configured to determine the magnitude of the current supplied to the LED (76a) by determining the first factor based on the cumulative lighting time acquired and multiplying a reference current value by the first factor determined.

8. The culture system (200) according to claim 6, wherein:
the control apparatus (100) is configured to store a second factor in association with the cumulative lighting time of the LED (76a), the second factor increasing monotonically or in a stepwise manner with an increase in the cumulative lighting time of the LED (76a), and
the control apparatus (100) is configured to determine the length of time for lighting of the LED (76a) by determining the second factor based on the cumulative lighting time acquired and multiplying a reference time length by the second factor determined.

9. The culture system (200) according to claim 1, wherein
the control apparatus (100) is configured to reduce a current supplied to the LED (76a) and to increase a time for lighting of the LED (76a) as a temperature inside the box (2) increases.

10. The culture system (200) according to claim 9, wherein:
the control apparatus (100) is configured to store a third factor in association with the temperature inside the box (2), the third factor decreasing monotonically or in a stepwise manner with an increase in the temperature inside the box (2), and
the control apparatus (100) is configured to determine a magnitude of the current supplied to the LED (76a) by determining the third factor based on the temperature inside the box (2) and multiplying a reference current value by the third factor determined.

11. The culture system (200) according to claim 10, wherein
the control apparatus (100) is configured to store a plurality of sets of the third factor and the temperature inside the box (2), and to select a set to be used for controlling the LED (76a) based on identification information for identifying at least one of the LED (76a) or the LED module (7).

12. The culture system (200) according to any one of claims 9 to 11, wherein:
the control apparatus (100) is configured to store a fourth factor in association with the temperature inside the box (2), the fourth factor increasing monotonically or in a stepwise manner with an increase in the temperature inside the box (2), and
the control apparatus (100) is configured to determine a length of time for lighting of the LED (76a) by determining the fourth factor based on the temperature inside the box (2) and multiplying a reference time length by the fourth factor determined.

13. The culture system (200) according to claim 12, wherein
the control apparatus (100) is configured to store a plurality of sets of the fourth factor and the temperature inside the box (2), and to select a set to be used for controlling the LED (76a) based on identification information for identifying at least one of the LED (76a) or the LED module (7).

## Patentansprüche

1. Ein Kultursystem (200), umfassend:
ein Leuchtdiodenmodul (LED-Modul) (7) beinhaltend:
eine LED (76a), die dazu eingerichtet ist, eine ultraviolette Strahlung zu emittieren,
eine Informationsspeichervorrichtung (77c), und
einen modulseitigen Anschluss (77d), der elektrisch mit der LED (76a) und der Informationsspeichervorrichtung (77c) verbunden ist; und
eine Kulturvorrichtung (1), beinhaltend:
eine Box (2),
einen vorrichtungsseitigen Anschluss (2e), der an der Box (2) angebracht ist und mit dem der modulseitige Anschluss (77d) verbunden ist,
eine Atmosphärenregelungsvorrichtung (5c, 8, 12a, 12b), die dazu eingerichtet ist, eine Atmosphäre im Inneren der Box (2) zu regeln, und
eine Steuervorrichtung (100), die dazu eingerichtet ist, die Atmosphärenregelungsvorrichtung (5c, 8, 12a, 12b) zu steuern,
**dadurch gekennzeichnet, dass**:
das LED-Modul (7) abnehmbar an der Kulturvorrichtung (1) befestigt werden kann, und
die Steuervorrichtung (100) dazu eingerichtet ist, die LED (76a) auf Grundlage von Information zu steuern, die von der Informationsspeichervorrichtung (77c) über den vorrichtungsseitigen Anschluss (2e) und den modulseitigen Anschluss (77d) erfasst wurden.

2. Das Kultursystem (200) nach Anspruch 1, wobei
die Information Identifikationsinformationen beinhaltet, die eine Identifizierung zumindest einer der LED (76a) oder des LED-Moduls (77d) ermöglichen.

3. Das Kultursystem (200) nach Anspruch 2, wobei
die Steuervorrichtung (100) dazu eingerichtet ist, auf Grundlage der erfassten Identifikationsinformationen zumindest eine von einer Länge einer Beleuchtungsdauer der LED (76a) oder einer Stärke eines der LED (76a) zugeführten Stroms zu steuern.

4. Das Kultursystem (200) nach einem der Ansprüche 1 bis 3, wobei
die Information eine kumulierte Leuchtdauer der LED (76a) beinhaltet.

5. Das Kultursystem (200) nach Anspruch 4, wobei
die Steuervorrichtung (100) dazu eingerichtet ist, auf Grundlage der erfassten kumulativen Leuchtdauer zumindest eine von einer Länge einer Beleuchtungsdauer der LED (76a) oder einer Stärke des der LED (76a) zugeführten Stroms zu steuern.

6. Das Kultursystem (200) nach Anspruch 5, wobei
die Steuervorrichtung (100) dazu eingerichtet ist, den der LED (76a) zugeführten Strom zu erhöhen oder die Beleuchtungsdauer der LED (76a) zu erhöhen, wenn die erfasste kumulative Leuchtdauer länger wird.

7. Das Kultursystem (200) nach Anspruch 6, wobei:
die Steuervorrichtung (100) dazu eingerichtet ist, einen ersten Faktor in Verbindung mit der kumulativen Leuchtdauer der LED (76a) zu speichern, wobei der erste Faktor monoton oder stufenweise mit einer Zunahme der kumulativen Leuchtdauer der LED (76a) ansteigt, und
die Steuervorrichtung (100) dazu eingerichtet ist, die Stärke des der LED (76a) zugeführten Stroms zu bestimmen, indem sie den ersten Faktor auf Grundlage der erfassten kumulativen Leuchtdauer ermittelt und einen Referenzstromwert mit dem ermittelten ersten Faktor multipliziert.

8. Das Kultursystem (200) nach Anspruch 6, wobei:
die Steuervorrichtung (100) dazu eingerichtet ist, einen zweiten Faktor in Verbindung mit der kumulativen Leuchtdauer der LED (76a) zu speichern, wobei der zweite Faktor monoton oder stufenweise mit einer Zunahme der kumulativen Leuchtdauer der LED (76a) ansteigt, und
die Steuervorrichtung (100) dazu eingerichtet ist, die Länge der Beleuchtungsdauer der LED (76a) zu bestimmen, indem sie den zweiten Faktor auf Grundlage der erfassten kumulativen Leuchtdauer ermittelt und eine Referenzzeitdauer mit dem ermittelten zweiten Faktor multipliziert.

9. Das Kultursystem (200) nach Anspruch 1, wobei
die Steuervorrichtung (100) dazu eingerichtet ist, einen der LED (76a) zugeführten Strom zu verringern und eine Beleuchtungsdauer der LED (76a) zu erhöhen, wenn die Temperatur im Inneren der Box (2) ansteigt.

10. Das Kultursystem (200) nach Anspruch 9, wobei:
die Steuervorrichtung (100) dazu eingerichtet ist, einen dritten Faktor in Verbindung mit der Temperatur im Inneren der Box (2) zu speichern, wobei der dritte Faktor mit steigender Temperatur im Inneren der Box (2) monoton oder stufenweise abnimmt, und
die Steuervorrichtung (100) dazu eingerichtet ist, eine Stärke des der LED (76a) zugeführten Stroms zu bestimmen, indem sie den dritten Faktor auf Grundlage der Temperatur im Inneren der Box (2) ermittelt und einen Referenzstromwert mit dem ermittelten dritten Faktor multipliziert.

11. Das Kultursystem (200) nach Anspruch 10, wobei
die Steuervorrichtung (100) dazu eingerichtet ist, eine Vielzahl von Sätzen des dritten Faktors und der Temperatur im Inneren der Box (2) zu speichern und einen für die Steuerung der LED (76a) zu verwendenden Satz auf Grundlage von Identifikationsinformationen zur Identifizierung zumindest einer von der LED (76a) oder des LED-Moduls (7) auszuwählen.

12. Das Kultursystem (200) nach einem der Ansprüche 9 bis 11, wobei:
die Steuervorrichtung (100) dazu eingerichtet ist, einen vierten Faktor in Verbindung mit der Temperatur im Inneren der Box (2) zu speichern, wobei der vierte Faktor monoton oder stufenweise mit einem Anstieg der Temperatur im Inneren der Box (2) zunimmt, und
die Steuervorrichtung (100) dazu eingerichtet ist, eine Länge der Beleuchtungsdauer der LED (76a) zu bestimmen, indem sie den vierten Faktor auf Grundlage der Temperatur im Inneren der Box (2) ermittelt und eine Referenzzeitdauer mit dem ermittelten vierten Faktor multipliziert.

13. Das Kultursystem (200) nach Anspruch 12, wobei
die Steuervorrichtung (100) dazu eingerichtet ist, eine Vielzahl von Sätzen des vierten Faktors und der Temperatur im Inneren der Box (2) zu speichern und einen für die Steuerung der LED (76a) zu verwendenden Satz auf Grundlage von Identifikationsinformationen zur Identifizierung zumindest einer von der LED (76a) oder des LED-Moduls (7) auszuwählen.

## Revendications

1. Système de culture (200), comprenant:
un module de diodes électroluminescentes (LED) (7) comprenant:
une LED (76a) configurée pour émettre un rayon ultraviolet,
un dispositif de stockage d'informations (77c), et
un connecteur côté module (77d) connecté électriquement à la LED (76a) et au dispositif de stockage d'informations (77c) ; et
un dispositif de culture (1) comprenant:
une boîte (2),
un connecteur côté appareil (2e) fixé au boîte (2) et auquel le connecteur côté module (77d) est connecté,
un dispositif de régulation de l'atmosphère (5c, 8, 12a, 12b) configuré pour réguler une atmosphère à l'intérieur de la boîte (2), et
un dispositif de commande (100) configuré pour commander le dispositif de régulation de l'atmosphère (5c, 8, 12a, 12b),
**caractérisé en ce que**:
le module LED (7) peut être fixé de manière amovible à le dispositive de culture (1), et
le dispositif de commande (100) est configuré pour commander la LED (76a) sur la base d'informations acquises à partir de le dispositive de stockage d'informations (77c) via le connecteur côté appareil (2e) et le connecteur côté module (77d).

2. Système de culture (200) selon la revendication 1, dans lequel
les informations comprennent des informations d'identification permettant d'identifier au moins l'une parmi la LED (76a) et le module LED (77d).

3. Système de culture (200) selon la revendication 2, dans lequel
sur la base des informations d'identification acquises, le dispositif de commande (100) est configuré pour commander au moins l'une parmi une durée d'éclairage de la LED (76a) et une intensité du courant fourni à la LED (76a).

4. Système de culture (200) selon l'une quelconque des revendications 1 à 3, dans lequel
ces informations comprennent la durée d'éclairage cumulée de la LED (76a).

5. Système de culture (200) selon la revendication 4, dans lequel
sur la base de la durée d'éclairage cumulée acquise, le dispositif de commande (100) est configuré pour commander au moins l'une parmi une durée d'éclairage de la LED (76a) ou une intensité d'un courant fourni à la LED (76a).

6. Système de culture (200) selon la revendication 5, dans lequel
le dispositif de commande (100) est configuré pour augmenter le courant fourni à la LED (76a) ou pour augmenter la durée d'éclairage de la LED (76a) à mesure que la durée d'éclairage cumulée enregistrée s'allonge.

7. Système de culture (200) selon la revendication 6, dans lequel:
le dispositif de commande (100) est configuré pour stocker un premier facteur en association avec la durée d'éclairage cumulée de la LED (76a), le premier facteur augmentant de manière monotone ou par paliers avec l'augmentation de la durée d'éclairage cumulée de la LED (76a), et
le dispositif de commande (100) est configuré pour déterminer l'intensité du courant fourni à la LED (76a) en déterminant le premier facteur sur la base de la durée d'éclairage cumulé acquise et en multipliant une valeur de courant de référence par le premier facteur déterminé.

8. Système de culture (200) selon la revendication 6, dans lequel:
le dispositive de commande (100) est configuré pour stocker un deuxième facteur en association avec la durée d'éclairage cumulé de la LED (76a), le deuxième facteur augmentant de manière monotone ou par paliers avec l'augmentation de la durée d'éclairage cumulé de la LED (76a), et
le dispositif de commande (100) est configuré pour déterminer la durée d'éclairage de la LED (76a) en déterminant le deuxième facteur sur la base de la durée d'éclairage cumulée acquise et en multipliant une durée de référence par le deuxième facteur déterminé.

9. Système de culture (200) selon la revendication 1, dans lequel
le dispositif de commande (100) est configuré pour réduire un courant fourni à la LED (76a) et pour augmenter une durée d'éclairage de la LED (76a) à mesure que la température à l'intérieur de la boîte (2) augmente.

10. Système de culture (200) selon la revendication 9, dans lequel:
le dispositif de commande (100) est configuré pour stocker un troisième facteur en association avec la température à l'intérieur de la boîte (2), le troisième facteur diminuant de manière monotone ou par paliers avec l'augmentation de la température à l'intérieur de la boîte (2), et
le dispositif de commande (100) est configuré pour déterminer une intensité du courant fourni à la LED (76a) en déterminant le troisième facteur sur la base de la température à l'intérieur de la boîte (2) et en multipliant une valeur de courant de référence par le troisième facteur déterminé.

11. Système de culture (200) selon la revendication 10, dans lequel
le dispositif de commande (100) est configuré pour stocker une pluralité d'ensembles du troisième facteur et de la température à l'intérieur de la boîte (2), et pour sélectionner un ensemble à utiliser pour commander la LED (76a) sur la base d'informations d'identification permettant d'identifier au moins l'une parmi la LED (76a) et le module LED (7).

12. Système de culture (200) selon l'une quelconque des revendications 9 à 11, dans lequel:
le dispositif de commande (100) est configuré pour stocker un quatrième facteur en association avec la température à l'intérieur de la boîte (2), le quatrième facteur augmentant de manière monotone ou par paliers avec l'augmentation de la température à l'intérieur de la boîte (2), et
le dispositif de commande (100) est configuré pour déterminer une durée d'éclairage de la LED (76a) en déterminant le quatrième facteur sur la base de la température à l'intérieur de la boîte (2) et en multipliant une durée de référence par le quatrième facteur déterminé.

13. Système de culture (200) selon la revendication 12, dans lequel
le dispositif de commande (100) est configuré pour stocker une pluralité d'ensembles comprenant le quatrième facteur et la température à l'intérieur de la boîte (2), et pour sélectionner un ensemble à utiliser pour commander la LED (76a) sur la base d'informations d'identification permettant d'identifier au moins l'une parmi la LED (76a) et le module de LED (7).
